# EUROPEAN PATENT SPECIFICATION

(11) **EP 4 010 483 B1**
(45) Date of publication and mention of the grant of the patent: **11.10.2023**
(21) Application number: 20749918.7
(22) Date of filing: 06.08.2020
(51) Int. Cl.: C12Q 1/04

(54) **DETECTION OF THE INHIBITION CAPACITY OF A FIRST MICROBIAL STRAIN ON THE GAS PRODUCTION OF A SECOND GAS PRODUCING MICROBIAL STRAIN**
DETEKTION DER HEMMUNGSKAPAZITÄT EINES ERSTEN MIKROBIELLEN STAMMS AUF DER GASPRODUKTION EINES ZWEITEN GASPRODUZIERENDEN MIKROBIELLEN STAMMS
DÉTECTION DE LA CAPACITÉ D'INHIBITION D'UNE PREMIÈRE SOUCHE MICROBIENNE SUR LA PRODUCTION DE GAZ D'UNE SECONDE SOUCHE MICROBIENNE PRODUCTRICE DE GAZ

(30) Priority: 09.08.2019 EP 19190970; 15.04.2020 EP 20169622
(43) Date of publication of application: 15.06.2022
(73) Proprietor: Chr. Hansen A/S, 2970 Hoersholm (DK)
(72) Inventor: COPANI, Giuseppe, 2970 Hoersholm (DK); SKJOET-RASMUSSEN, Line, 2970 Hoersholm (DK); MILORA, Nina, 2970 Hoersholm (DK); SEGURA, Audrey, 2970 Hoersholm (DK)
(86) International application number: PCT/EP2020/072142
(87) International publication number: WO 2021/028311

(56) References cited:
- WO-A1-2018/167171
- WO-A1-2018/226521
- SCHOSTER A ET AL: "In vitro inhibition of Clostridium difficile and Clostridium perfringens by commercial probiotic strains", ANAEROBE,, vol. 20, 1 April 2013 (2013-04-01), pages 36-41, XP002776546, DOI: 10.1016/J.ANAEROBE.2013.02.006
- V. URUBSCHUROV ET AL: "Porcine intestinal yeast species, Kazachstania slooffiae , a new potential protein source with favourable amino acid composition for animals", JOURNAL OF ANIMAL PHYSIOLOGY AND ANIMAL NUTRITION., vol. 102, no. 2, 1 April 2018 (2018-04-01) , pages e892-e901, XP055513535, DE ISSN: 0931-2439, DOI: 10.1111/jpn.12853

## Description

### FIELD OF THE INVENTION

The present invention relates to a method for evaluating and/or quantifying the inhibition capacity of at least one, potentially inhibiting, first microbial strain towards at least one gas producing, potentially undesirable, second microbial strain.

### BACKGROUND OF THE INVENTION

A number of gas producing microbial strains are undesirable. For example, *Clostridium perfringens* type A, *Clostridium perfringens* type C, and *Clostridium septicum* are toxin-producing and involved in many diseases. *Clostridium perfringens* type A is involved in myonecrosis (gas-gangrene) in humans, enterotoxemia of lambs, cattle and goats, and necrotic enteritis in poultry, and *Clostridium perfringens* type C is involved in "struck" in sheep, enterotoxemia in lambs and pigs, and necrotic enteritis in poultry (Hatheway 2016 Toxigenic clostridia. Clin Microbiol Rev 2016;3:66-98).

Potentially undesirable microbial strains such as *Clostridium* spp. may be inhibited by other microbial strains.

The capacity of a microbial strain to inhibit another, potentially undesirable, microbial strain is conventionally evaluated *in vitro* by co-culture of the two microbial strains on a solid medium and assessment of the presence or absence of inhibition zones. This method was for example used in WO 2018/167171 to assess the inhibitory activity of the *Bacillus subtilis* strains deposited as DSM32324 and DSM32325 against *Escherichia coli* and *Clostridium perfringens.* However, only a qualitative evaluation of inhibition can be made based on this method. Moreover, the method has low sensitivity and it can only be used with strains that grow in a uniform way on agar media. Some strains, such as *Clostridium septicum,* cannot easily be counted because they do not grow in distinct colonies. The conventional methods of evaluating inhibition capacity also suffer from being quite time consuming and do not take the difficulties in cultivating anaerobic microbial strains into account. For instance, the conventional methods are usually not applicable to obligate anaerobic microbial strains since some O₂ exposure cannot be prevented with these methods.

WO2018/226521 describes methods and compositions for the reduction of intestinal gas/flatulence. Example 1 describes a test system wherein an inverted Durham tube, a small inverted tube which can serve as a trap for gas bubbles generated during fermentation, is placed in a test tube with 10 ml of media. The gas production is evaluated by visual inspection in a qualitative manner: - no gas production, + minimal gas production (small bubble in Durham tube), ++ substantial amount of gas production (half Durham tube filled with gas bubble), and +++ excess amount of gas production (almost 90% of Durham tube filled with gas bubble). No inoculation dose of the bacteria is provided. The patent application neither provides information about when the gas production is evaluated, i.e. the incubation time of the experiment. Thus, the method described in Example 1 is not reproducible.

Hence, there exists a need for a time-efficient, reliable and quantitative method for assessing the capacity of at least one first microbial strain to inhibit at least one gas producing, potentially undesirable, second microbial strain. In particular, an automated and quantitative method is needed which is standardized and reproducible and can distinguish between the efficiency of individual bacterial strains to control growth of gas producing strains in a manner not left to human subjective evaluation.

### SUMMARY OF THE INVENTION

The present inventors have developed a method that can be used to evaluate, in a quantitative manner, the inhibition efficiency of microbial strains. This method is based on the finding that, when a gas producing microbial strain is cultivated in the presence of a microbial strain capable of inhibition of said gas producing microbial strain, the total gas produced by the gas producing microbial strain is reduced. Hence, by determining the reduction in total gas production it is possible to qualitatively and quantitatively assess the inhibition capacity of the microbial strain on a gas producing, potentially undesirable, microbial strain.

Moreover, the present method allows for conducting dose-response trials as well as for testing several microbial strains independently or simultaneously.

The present method can also be used as a medium- or high-throughput assay for screening the inhibition capacity of known or new microbial strains toward gas producing, potentially undesirable, microbial strains.

Thus, it is an aspect of the present disclosure to provide a method for quantifying the inhibition of at least one first microbial strain on the gas production of at least one gas producing second microbial strain, the method comprising:
a) providing at least one first microbial strain;
b) providing at least one gas producing second microbial strain;
c) cultivating the at least one first microbial strain and the at least one gas producing second microbial strain in a closed culture system;
d) determining the volume of the total gas produced in the closed culture system of c);
e) determining the volume of the total gas produced by the at least one gas producing second microbial strain when cultivated in absence of any further microbial strains in a closed culture system; and
f) comparing the volume of the total gas of d) and e).

More specifically, the present invention provides a method for quantifying the inhibition efficiency of at least one first bacterial or yeast strain on a single gas producing second bacterial or yeast strain, the method comprising:
a) providing at least one first bacterial or yeast strain which produces no or only negligible volumes of gas;
b) providing a single gas producing second bacterial or yeast strain;
c) cultivating the at least one first bacterial or yeast strain and the single gas producing second bacterial or yeast strain in a closed culture system which is gas impermeable and to which no additional nutrients are added and waste products are not removed;
d) measuring the volume of the total gas produced in the closed culture system of c) over a period of time by means of an automatic gas measuring device measuring the pressure of the gas produced;
e) measuring the volume of the total gas produced by the single gas producing second bacterial or yeast strain when cultivated in absence of any further bacterial or yeast strains in a closed culture system over the same period of time by means of an automatic gas measuring device measuring the pressure of the gas produced; and
f) comparing the pressure of the total gas of d) and e).

In one embodiment of the present disclosure when the pressure of the total gas of d) is lower than the pressure of the total gas of e), the at least one first bacterial or yeast strain inhibits the gas producing second bacterial or yeast strain.

In one embodiment of the present disclosure when the pressure of the total gas of d) is equal to or higher than the pressure of the total gas of e), the at least one first microbial strain does not inhibit the gas producing second microbial strain.

By the use of an automatic gas measuring device a method is provided which can be standardized and makes it possible to quantify the inhibition efficiency of individual strains to control growth of gas producing strains in a reproducible manner.

In contrast to the prior art the method of the present invention makes it possible to differentiate between the inhibitory efficiency of individual strains even if the difference is small and to calculate whether the difference is statistically significant even for small differences not detectable by visual inspection.

In one embodiment, the methods of the present disclosure are conducted *in vitro.* For example, the methods of the present disclosure may comprise measuring and determining the *in vitro* gas production of at least one gas producing second microbial strain when cultivated in presence of at least one, potentially inhibiting, first microbial strain.

A further aspect of the present disclosure is to provide a use of a gas measuring system for detecting and/or quantifying the inhibitory effect of at least one first microbial strain on at least one gas producing second microbial strain.

An even further aspect of the present disclosure is to provide a use of a gas measuring system for identifying a first microbial strain capable of inhibiting at least one gas producing second microbial strain.

In one embodiment of the present disclosure the gas measuring system comprises:
a) vials suitable for cultivation of one or more microbial strains in a closed culture system;
b) a gas measuring device; and
c) means for determining the total volume of gas produced in the closed culture system.

Vials suitable for cultivation of one or more microbial strains in a closed culture system are known in the art. Plastic vials or glass vials, which are gas impermeable, are suitable.

Gas measuring devices are also known in the art. Automatic gas measuring devices are suitable.

Means for determining the total volume of gas produced in the closed culture system are also known in the art and depend on the gas measuring device used. For example, automatic gas measuring devices may also be capable of determining the total volume of gas.

### DEFINITIONS

A "microbial strain" as used herein refers to a microorganism which remains genetically unchanged when grown or multiplied. The multiplicity of identical microorganisms is included. Examples of microorganisms which are within the scope of this invention are bacteria and yeast whereas anaerobic fungi, protozoa and bacteriophages are considered outside the scope of the present invention

"A gas producing microbial strain" as used herein refers to a microbial strain which is producing noticeable amounts of gas during metabolism. Gas producing microbial strains are usually microbial strains which employ fermentation as a means of producing energy for their growth. Some clostridia, fusobacteria, *Escherichia coli* and *Salmonella* are examples of gas producing bacteria. Examples of other gas producing microorganisms are yeast such as *Saccharomyces* and *Pichia,* e.g. *Pichia fermentans, Pichia canadensis,* and *Pichia anomala.*

"Closed culture system" as used herein refers to a device that is used to propagate microorganisms of at least one microbial strain. Such a system should permit at least one cell division to occur and be gas impermeable. Moreover, during a cultivation batch in a closed culture system no additional nutrients are added to the system and waste products are not removed. Cultures in a closed system usually follow a predicted growth curve.

"Volume of total gas" as used herein refers to the total cumulative gas produced during a cultivation batch of at least one gas producing microbial strain in a closed culture system. The volume of total gas is a sum of the volumes of all gases produced during cultivation, wherein said gases usually are any of carbon dioxide, carbon monoxide, methane, nitrogen, oxygen and other gases. The volume of total gas can be measured in for example ml, cm³ or other volume units or in pressure units for example psi. For ease of understanding, the invention is described by use of the term "volume of total gas". However, as evident from the above this term to be construed to cover both the volume of the total gas and the pressure of the total gas unless otherwise indicated herein or clearly contradicted by context.

"Inhibition" as used herein refers to a decrease in growth of a microbial strain compared to growth of said microbial strain under different culture conditions, such as a decrease in growth of a microbial strain when grown in combination with at least one other microbial strain compared to the growth of the same strain when grown alone as a single strain.

Different parameters can be monitored to detect inhibition of a microbial strain depending on the metabolism of said microbial strain. Turbidity of the culture and presence of inhibition zones are typically used to determine whether or not there is inhibition in liquid cultures and solid cultures, respectively. In the present disclosure, the volume of total gas produced in a closed culture system has been used as a sensitive and precise way to monitor growth of gas producing microbial strain or strains and this parameter has be used as a means of measuring inhibition.

The term "probiotic microbial strain" refers to a culture of live or freeze-dried microorganisms, dead microorganisms, fragments of microorganisms and extracts or supernatants of microorganisms which, when applied to animal or human, beneficially affects the host (FAO/WHO (2001) Health and Nutritional Properties of Probiotics in Food including Powder Milk with Live Lactic Acid Bacteria. Report of a Joint FAO/WHO Expert Consultation on Evaluation of Health and Nutritional Properties of Probiotics in Food Including Powder Milk with Live Lactic Acid Bacteria). Probiotic microbial strains can be administered as Direct-Fed Microbial (DFM).

The term Direct-Fed Microbial" or "DFM" means live microorganisms including spores which, when administered in adequate amounts, confer a benefit, such as improved digestion or health, on the host.

### DETAILED DISCLOSURE OF THE INVENTION

In one aspect of the present disclosure a method is provided for detecting the inhibition of at least one first microbial strain on at least one gas producing second microbial strain, the method comprising:
a) providing at least one first microbial strain;
b) providing at least one gas producing second microbial strain;
c) cultivating the at least one first microbial strain and the at least one gas producing second microbial strain in a closed culture system;
d) determining the volume of the total gas produced in the closed culture system of c);
e) determining the volume of the total gas produced by the at least one gas producing second microbial strain when cultivated in absence of any further microbial strains in a closed culture system; and
f) comparing the volume of the total gas of d) and e).

In one aspect of the present disclosure a method is provided for quantifying the inhibition of at least one first microbial strain on at least one gas producing second microbial strain, the method comprising:
a) providing at least one first microbial strain;
b) providing at least one gas producing second microbial strain;
c) cultivating the at least one first microbial strain and the at least one gas producing second microbial strain in a closed culture system;
d) determining the volume of the total gas produced in the closed culture system of c);
e) determining the volume of the total gas produced by the at least one gas producing second microbial strain when cultivated in absence of any further microbial strains in a closed culture system; and
f) comparing the volume of the total gas of d) and e).

In one embodiment of the present disclosure the methods of the present disclosure are conducted with one potentially inhibiting first microbial strain and one gas producing second microbial strain. Evident to a person of ordinary skill in the art, the methods of the present disclosure may be performed with one, two, three, four, five, six, seven, eight, nine, ten or even more first microbial strains combined with one, two, three, four, five, six, seven, eight, nine, or ten or even more second microbial strains. Importantly, the number of microbial strains will be known and definite in contrast to a biological sample where the number of strains is unknown and much higher.

In one embodiment of the present disclosure the at least one first microbial strain is a single microbial strain.

In one embodiment of the present disclosure the at least one first microbial strain is two or more microbial strains. For example, the inhibition capacity of a combination of two microbial strains on one gas producing second microbial strain may be determined, a combination of three microbial strains on one gas producing second microbial strain may be determined, a combination of four microbial strains on one gas producing second microbial strain may be determined, a combination of five microbial strains on one gas producing second microbial strain may be determined, a combination of six microbial strains on one gas producing second microbial strain may be determined, a combination of seven microbial strains on one gas producing second microbial strain may be determined, a combination of eight microbial strains on one gas producing second microbial strain may be determined, a combination of nine microbial strains on one gas producing second microbial strain may be determined, or a combination often microbial strains on one gas producing second microbial strain may be determined. This may be useful as some microbial strains, all falling within the definition of a first microbial strain, may inhibit gas producing microbial strains to a larger extent when present in combination compared to if present as single strain.

In one embodiment of the present disclosure the at least one second microbial strain is a single microbial strain.

In one embodiment of the present disclosure the at least one second microbial strain is two or more microbial strains. For example, the inhibition capacity of a first microbial strain may be determined on one gas producing second microbial strain, or a combination of two gas producing second microbial strains, such as of a combination of three gas producing second microbial strains, such as of a combination of four gas producing second microbial strains, such as of a combination of five gas producing second microbial strains. This may be useful as some gas producing second microbial strains, all falling within the definition of a second microbial strain, may be present simultaneously *in vivo.*

For ease of understanding, the invention is in the following described by use of the terms "a" and "an" and "the" rather than "at least one". However, in the context of describing the invention these terms are to be construed to cover both the singular and the plural unless otherwise indicated herein or clearly contradicted by context.

The gas production capacity of a gas producing microbial strain can be determined in terms of volume of total gas. The volume of total gas produced during cultivation of a microbial strain can be determined by measuring the gas production over a period of time, e.g. at regular intervals during cultivation, or by measuring the accumulated gas at the end of the cultivation period. If the gas production is measured during the cultivation, the time intervals between the individual measurements can be selected to provide the desired precision e.g. to provide an almost continuous measurement. When the gas production is measured in this manner, it is possible to go back to any desired time point even after the experiment.

In one embodiment of the present invention the cumulative gas production is measured during the cultivation for a period of time of 4 hours of cultivation in the closed culture system, such as for a period of time of 8 hours of cultivation, such as for a period of time of 12 hours of cultivation, such as for a period of time of 18 hours of cultivation, such as for a period of time of 24 hours of cultivation, such as for a period of time of 30 hours of cultivation, such as for a period of time of 36 hours of cultivation, such as for a period of time of 42 hours of cultivation, such as for a period of time of 48 hours of cultivation, such as for a period of time of 54 hours of cultivation, such as for a period of time of 60 hours of cultivation, such as for a period of time of 66 hours of cultivation, such as for a period of time of 72 hours of cultivation.

In order to determine the volume of total gas produced by one gas producing microbial strain, said gas producing microbial strain should be cultivated as a single strain in a closed culture system.

The present disclosure provides a method for determining whether a first microbial strain inhibits a gas producing second microbial strain by cultivating said first and second microbial strains together in the same closed culture system and measuring the volume of total gas produced during cultivation of the first and second microbial strains. Provided that the volume of total gas produced by the gas producing second microbial strain when cultivated in the closed culture system as a single strain is known, it will be possible to assess and quantify the inhibition capacity of the first microbial strain against the gas producing second microbial strain by comparing:
- the volume of total gas produced when the gas producing second microbial strain is cultivated as a single strain, i.e. in absence of other microbial strains; to
- the volume of total gas produced when the gas producing second microbial strain is cultivated together with the potentially inhibiting first microbial strain.

Co-cultivation of a gas producing microbial strain in presence of an inhibiting microbial strain will cause a decrease in gas production which can be determined by the person of skill in the art.

In one embodiment of the present disclosure when the volume of the total gas of d) is lower than the volume of the total gas of e), the first microbial strain is capable of inhibiting the gas producing second microbial strain. If desired, the inhibition of the gas producing second microbial strain by the first microbial strain can be quantified.

In one embodiment of the present disclosure when the volume of the total gas of d) is equal to or higher than the volume of the total gas of e), the first microbial strain is not capable of inhibiting the gas producing second microbial strain.

In one embodiment of the present disclosure determining the volume of the total gas of e) is conducted prior to determining the volume of the total gas of d). For example, the volume of the total gas of e) may already be known at the time of determining the volume of the total gas of d). In such cases, step e) may be omitted from the methods as disclosed herein and the total volume of gas of d) may be compared to such a predetermined gas volume to determine the inhibitory capacity of the first microbial strain on the gas producing second microbial strain.

In one embodiment of the present disclosure determining the volume of the total gas of e) is conducted simultaneously to determining the volume of the total gas of d). For example, a series of tests are conducted in parallel to assess the total gas production of a gas producing second microbial strain when cultivated as a single strain and when cultivated together with a first microbial strain.

In one embodiment of the present disclosure determining the volume of the total gas of e) is conducted subsequently to determining the volume of the total gas of d). For example, the volume of the total gas of e) is known first after determining the volume of the total gas of d).

In one embodiment of the present disclosure determining the volume of the total gas is conducted by means of an automated gas production system. Several automated gas production systems can be used and are commercially available, for example ANKOM RF Gas Production System (ANKOM Technology, US), Fermograph (ATTO Corporation, JP), AMPTS II, µFLOW and related products (Bioprocess Control, SE).

In one embodiment of the present disclosure the total gas produced is the cumulative gas production after at least 4 hours of cultivation in the closed culture system, such as after at least 8 hours of cultivation, such as after at least 12 hours of cultivation, such as after at least 18 hours of cultivation, such as after at least 24 hours of cultivation, such as after at least 30 hours of cultivation, such as after at least 36 hours of cultivation, such as after at least 42 hours of cultivation, such as after at least 48 hours of cultivation, such as after at least 54 hours of cultivation, such as after at least 60 hours of cultivation, such as after at least 66 hours of cultivation, such as after at least 72 hours of cultivation. For example, the total gas produced is the cumulative gas production as measured at the end of the cultivation which is when the gas producing second microbial strain no longer grows.

In one embodiment of the present disclosure the growth of the gas producing second microbial strain is measured and determined in parallel with determination of the total gas production in the closed culture system.

In one embodiment of the present disclosure the growth curve as well as the total cultivation time of the gas producing second microbial strain is known.

In one embodiment of the present disclosure the closed culture system is a liquid culture system. Liquid may advantageously be used for cultivating gas producing microbial strains which do not easily grow on solid media.

In one embodiment of the present disclosure the closed culture system is an anaerobic culture system. Anaerobic cultivation may advantageously be used for detecting and/or quantifying the inhibition on anaerobic gas producing microbial strains.

In one embodiment of the present disclosure the method further comprises calculating the percentage of inhibition of the first microbial strain on the gas producing second microbial strain. The percentage of inhibition may for example be calculated based on an algorithm that takes into account the volume of total gas produced by the gas producing second microbial strain when cultivated as single strain and when cultivated in presence of the potentially inhibiting first microbial strain.

It is generally considered advantageous that the gas producing strain is inhibited to a substantial extent such as at least 50%, such as at least 66%, such as at least 75%, such as at least 80%, such as at least 85%, such as at least 90%, e.g. at least 95%.

The method of the present invention makes it possible to differentiate between the inhibitory efficiency of individual strains even if the difference is small and to calculate whether the difference is statistically significant even for small differences. Strains may be ranked on the basis of their inhibitory efficiency on one or more gas producing strains.

In one embodiment of the present disclosure the method further comprises comparing the inhibitory efficiency of at least two first bacterial or yeast strains with regard to their inhibitory efficiency towards the same gas producing second bacterial or yeast strain.

The present disclosure also relates to methods for screening and evaluating a number of microbial strains for their capacity to inhibit a gas producing microbial strain. Although screening methods having the same purpose exist the methods of the present disclosure provide several advantages over previously known methods which are largely based on the formation of inhibition zones on agar plates. The methods of the present disclosure allow for a precise quantification of the inhibition thanks to ease in determining the volume of total gas produced during cultivation in a closed culture system. Moreover, the methods of the present disclosure allow for screening against microbial strains which do not grow well on solid media, which cannot be counted after cultivation, or which do not grow uniformly but form filaments instead.

In one embodiment, the present disclosure relates to a medium- or high-throughput assay for screening the inhibition capacity of microorganisms of a first, potentially inhibiting, microbial strain toward a gas producing, potentially undesirable, second microbial strain.

For the present method to correctly quantify the inhibition of a first microbial strain on a gas producing second microbial strain it is important that the volume of total gas produced by the first microbial strain is negligible compared to the volume of total gas produced by the gas producing second microbial strain.

Hence, in one embodiment of the present disclosure the first microbial strain produces no or only negligible volumes of gas.

In one embodiment of the present disclosure the first microbial strain is not a gas producing microbial strain.

In one embodiment of the present disclosure the first microbial strain is capable of inhibiting a gas producing microbial strain.

In one embodiment of the present disclosure the first microbial strain is a probiotic microbial strain.

Microbial strains are generally classified as aerobic or anaerobic strains. Some aerobic strains are also able to grow in absence of oxygen, i.e. they are not strict aerobic.

In one embodiment of the present disclosure the first microbial strain is aerobic and said strain is facultative anaerobic, microaerophilic or aerotolerant.

In one embodiment of the present disclosure the first microbial strain is anaerobic, and said strain is anaerobic, facultative anaerobic, aerotolerant or obligate anaerobic.

In one embodiment of the present disclosure the first microbial strain is a bacterial strain.

In one embodiment of the present disclosure the first microbial strain is of the genus *Bacillus,* such as of the species *Bacillus altitudinis, Bacillus amyloliquefaciens,* e.g. *Bacillus amyloliquefaciens* subsp. *amyloliquefaciens* or *Bacillus amyloliquefaciens* subsp. *plantarum, Bacillus atrophaeus, Bacillus licheniformis, Bacillus megaterium, Bacillus methylotrophicus, Bacillus mojavensis, Bacillus pumilus, Bacillus safensis, Bacillus simplex, Bacillus stratosphericus, Bacillus subtilis, Bacillus siamensis, Bacillus vallismortis, Bacillus velezensis,* or *Bacillus tequilensis.*

In one embodiment of the present disclosure the first microbial strain is a lactic acid bacterial strain.

In one embodiment of the present disclosure the first microbial strain is of the genus *Lactobacillus,* such as *Lactobacillus acidophilus, Lactobacillus animalis, Lactobacillus brevis, Lactobacillus buchneri, Lactobacillus casei, Lactobacillus delbrueckii, Lactobacillus diolivorans, Lactobacillus fermentum, Lactobacillus gasseri, Lactobacillus johnsonii, Lactobacillus plantarum, Lactobacillus paracasei, Lactobacillus reuteri,* and *Lactobacillus rhamnosus.*

In one embodiment of the present disclosure the first microbial strain is of the genus *Lactococcus,* such as *Lactococcus lactis.*

In one embodiment of the present disclosure the first microbial strain is of the genus *Enterococcus,* such as *Enterococcus faecium.*

In one embodiment of the present disclosure the first microbial strain is of the genus *Escherichia,* e.g. bacteria of the species *Escherichia coli,* an example of which is *Escherichia coli* Nissle 1917.

In one embodiment of the present disclosure the first microbial strain is of the genus *Pediococcus,* such as *Pediococcus pentasaceus* or *Pediococcus acidilactici.*

In one embodiment of the present disclosure the first microbial strain is of the genus *Propionibacterium,* such as *Propionibacterium freudenreichii* or *Propionibacterium acidipropionici.*

In one embodiment of the present disclosure the first microbial strain is of the genus *Bifidobacterium,* such as *Bifidobacterium animalis, Bifidobacterium breve, Bifidobacterium infantis,* or *Bifidobacterium longum.*

In one embodiment of the present disclosure the first microbial strain is selected from the group consisting of:
a) the *Bacillus amyloliquefaciens* strain deposited as DSM25840,
b) the *Bacillus subtilis* strain deposited as DSM32324,
c) the *Bacillus subtilis* strain deposited as DSM32325,
d) the *Bacillus subtilis* strain deposited as DSM17231,
e) the *Bacillus licheniformis* strain deposited as DSM17236,
f) the *Lactobacillus animalis* strain deposited as PTA-6750, and
g) the *Propionibacterium freudenreichii* strain deposited as PTA-6752.

In one embodiment the at least one microbial strain is two bacterial strains, the *Bacillus licheniformis* strain deposited as DSM17236 and the *Bacillus subtilis* strain deposited as DSM17231.

In one embodiment the at least one microbial strain is two bacterial strains, the *Lactobacillus animalis* strain deposited as PTA-6750 and the *Propionibacterium freudenreichii* strain deposited as PTA-6752.

In one embodiment the at least one microbial strain is three bacterial strains, the *Bacillus subtilis* strain deposited as DSM32324, the *Bacillus subtilis* strain deposited as DSM32325, and the *Bacillus amyloliquefaciens* strain deposited as DSM25840.

In one embodiment of the present disclosure the gas producing second microbial strain is a pathogenic microbial strain.

In one embodiment of the present disclosure the gas producing second microbial strain is a bacterial strain.

In one embodiment of the present disclosure the gas producing second microbial strain is capable of anaerobic fermentation. For example, the second microbial strain is capable of producing gas when cultivated anaerobically in a closed culture system.

In one embodiment of the present disclosure the gas producing second microbial strain is anaerobic.

In one embodiment of the present disclosure the gas producing second microbial strain is anaerobic, and said strain is anaerobic, facultative anaerobic, aerotolerant or obligate anaerobic.

In one embodiment of the present disclosure the gas producing second microbial strain is of the genus *Clostridium.*

In one embodiment of the present disclosure the gas producing second microbial strain is of the species *Clostridium perfringens* or *Clostridium septicum.*

In one embodiment of the present disclosure the gas producing second microbial strain is of the species *Clostridium septicum* and the at least one microbial strain is three bacterial strains, the *Bacillus subtilis* strain deposited as DSM32324, the *Bacillus subtilis* strain deposited as DSM32325, and the *Bacillus amyloliquefaciens* strain deposited as DSM25840.

In one embodiment of the present disclosure the gas producing second microbial strain is of the species *Clostridium perfringens* type A.

In one embodiment of the present disclosure the gas producing second microbial strain is of the species *Clostridium perfringens* type A and the at least one microbial strain is two bacterial strains, the *Bacillus licheniformis* strain deposited as DSM17236 and the *Bacillus subtilis* strain deposited as DSM17231.

In one embodiment of the present disclosure the gas producing second microbial strain is of the species *Clostridium perfringens* type A and the at least one microbial strain is two bacterial strains, the *Lactobacillus animalis* strain deposited as PTA-6750 and the *Propionibacterium freudenreichii* strain deposited as PTA-6752.

In one embodiment of the present disclosure the gas producing second microbial strain is of the species *Clostridium perfringens* type C.

In one embodiment of the present disclosure the gas producing second microbial strain is of the species *Clostridium perfringens* type C and the at least one microbial strain is two bacterial strains, the *Bacillus licheniformis* strain deposited as DSM17236 and the *Bacillus subtilis* strain deposited as DSM17231.

In one embodiment of the present disclosure the gas producing second microbial strain is of the species *Clostridium perfringens* type C and the at least one microbial strain is two bacterial strains, the *Lactobacillus animalis* strain deposited as PTA-6750 and the *Propionibacterium freudenreichii* strain deposited as PTA-6752.

In one embodiment of the present disclosure the gas producing second microbial strain is of the phylum fusobacteria or genus *Escherichia* such as *Escherichia coli,* or *Salmonella.*

In one embodiment of the present disclosure the gas producing second microbial strain is of the genus *Saccharomyces.*

In one embodiment of the present disclosure, the gas producing second microbial strain is of the genus *Pichia,* such as *Pichia fermentans, Pichia canadensis,* and *Pichia anomala.*

The use of the terms "a" and "an" and "the" and similar referents in the context of describing the invention (especially in the context of the following claims) are to be construed to cover both the singular and the plural unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### LEGEND TO FIGURES

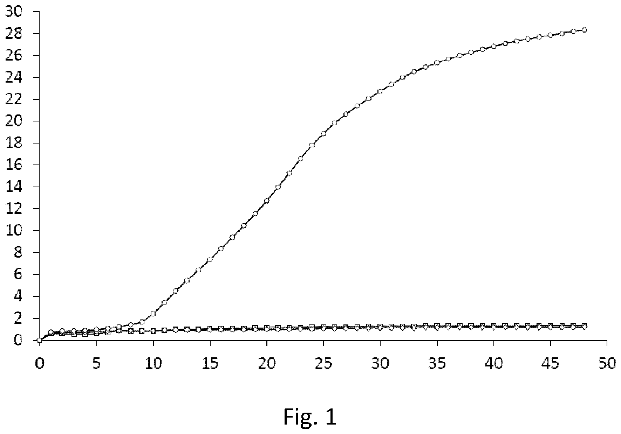

Fig. 1: Cumulative pressure graph of *Bacillus subtilis* strain deposited as DSM32324 ( 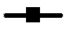 ), *Bacillus subtilis* strain deposited as DSM32325 ( 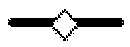 ), and *Clostridium septicum* strain deposited as DSM7534 ( 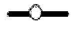 ) during 48 hours incubation. On the x-axis is time (hours) and on the y-axis is pressure (psi).

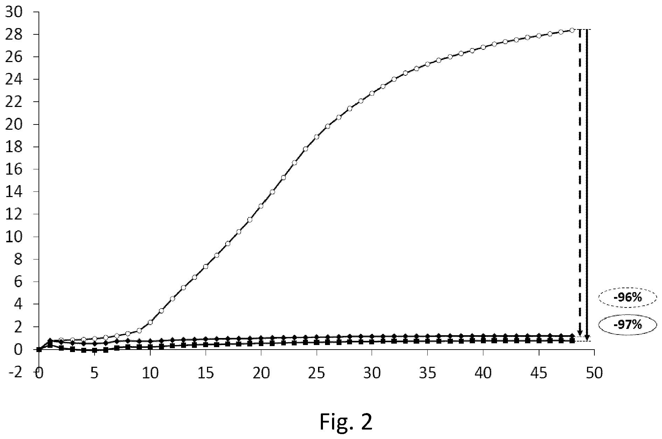

Fig. 2: Cumulative pressure graph of *Clostridium septicum* strain deposited as DSM7534 during 48 hours incubation as single strain ( 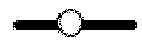 ), together with *Bacillus subtilis* strain deposited as DSM32324 ( 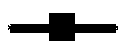 ), and together with *Bacillus subtilis* strain deposited as DSM32325 ( 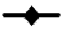 ). On the x-axis is time (hours) and on the y-axis is pressure (psi).

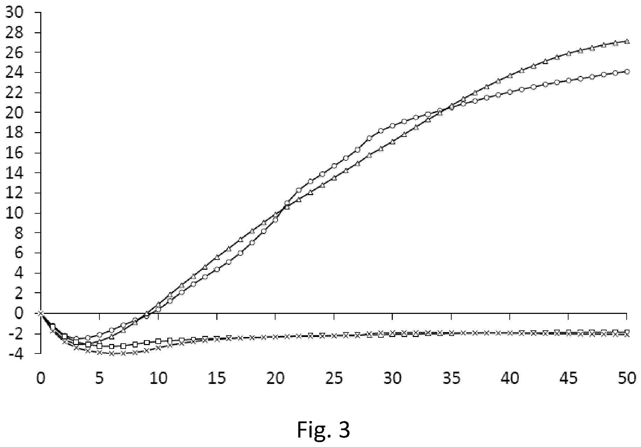

Fig. 3: Cumulative pressure graph of *Bacillus subtilis* strain deposited as DSM32324 ( 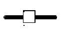 ), *Bacillus licheniformis* strain deposited as DSM17236 ( 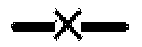 ), *Clostridium septicum* strain deposited as DSM7534 ( 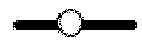 ) and *Clostridium septicum* Strain D ( 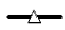 ) during 48 hours incubation. On the x-axis is time (hours) and on the y-axis is pressure (psi).

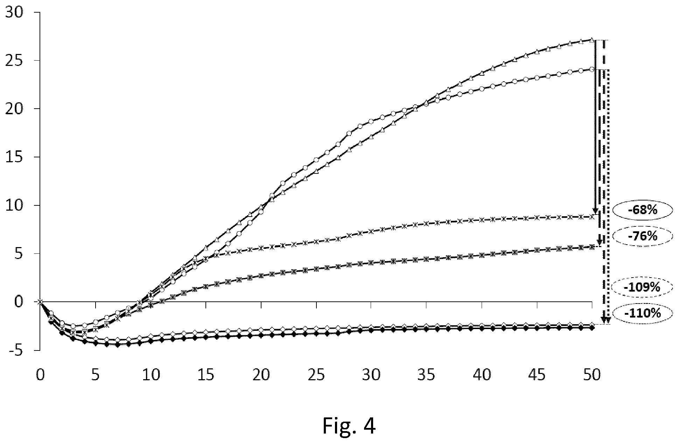

Fig. 4: Cumulative pressure graph of *Clostridium septicum* strain deposited as DSM7534 during 48 hours incubation as single strain ( 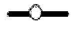 ), together with *Bacillus subtilis* strain deposited as DSM32324 ( 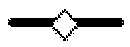 ), and together with *Bacillus licheniformis* strain deposited as DSM17236 ( 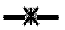 ). Cumulative pressure graph of *Clostridium septicum* strain D during 48 hours incubation as single strain ( 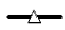 ), together with *Bacillus subtilis* strain deposited as DSM32324 ( 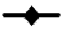 ), and together with *Bacillus licheniformis* strain deposited as DSM17236 ( 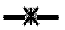 ). On the x-axis is time (hours) and on the y-axis is pressure (psi).

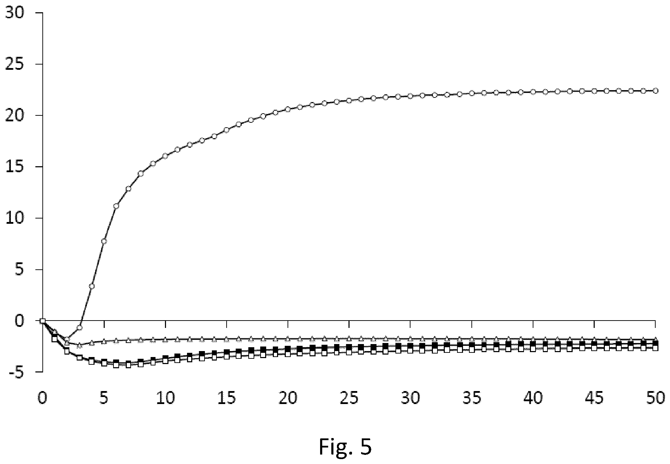

Fig. 5: Cumulative pressure graph of *Bacillus subtilis* strain deposited as DSM32324 ( 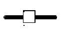 ), *Bacillus subtilis* strain A ( 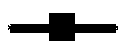 ), *Lactobacillus animalis* strain deposited as PTA-6750 ( 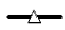 ), and *Clostridium perfringens* type A strain deposited as DSM756 ( 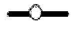 ) during 48 hours incubation. On the x-axis is time (hours) and on the y-axis is pressure (psi).

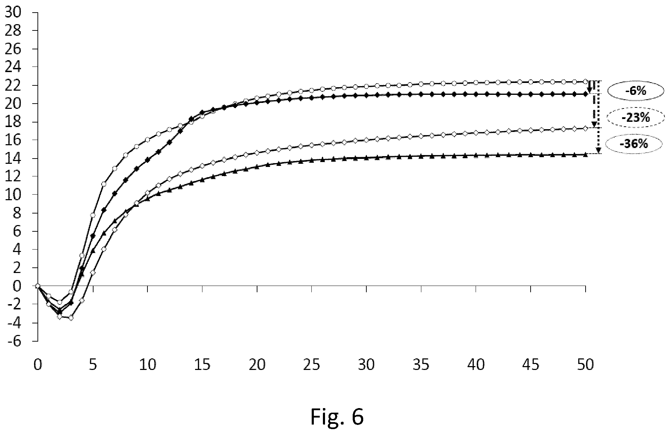

Fig. 6: Cumulative pressure graph of *Clostridium perfringens* type A strain deposited as DSM756 during 48 hours incubation as single strain ( 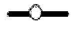 ), together with *Bacillus subtilis* strain deposited as DSM32324 ( 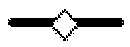 ), together with *Bacillus subtilis* strain A ( 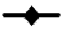 ), and together with *Lactobacillus animalis* strain deposited as PTA-6750 ( 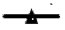 ). On the x-axis is time (hours) and on the y-axis is pressure (psi).

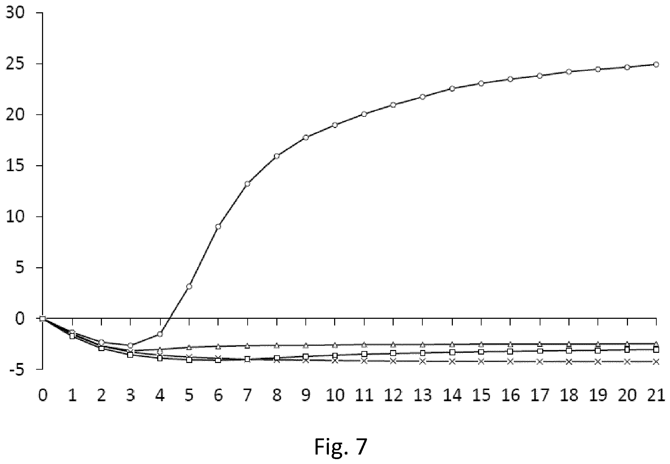

Fig. 7: Cumulative pressure graph of *Bacillus subtilis* strain deposited as DSM32324 ( 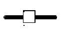 ), *Lactobacillus animalis* strain deposited as PTA-6750 ( 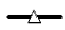 ), *Propionibacterium freudenreichii* Strain B ( 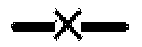 ), and *Clostridium perfringens* type C strain deposited as NCTC3180 ( 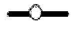 ) during 48 hours incubation. On the x-axis is time (hours) and on the y-axis is pressure (psi).

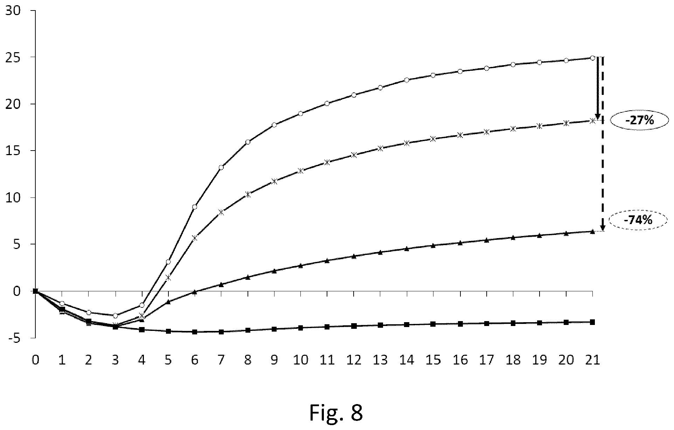

Fig. 8: Cumulative pressure graph of *Clostridium perfringens* type C strain deposited as NCTC3180 during 48 hours incubation as single strain ( 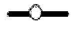 ), together with *Bacillus subtilis* strain deposited as DSM32324 ( 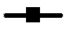 ), together with *Propionibacterium freudenreichii* Strain B ( 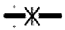 ), and together with *Lactobacillus animalis* strain deposited as PTA-6750 ( 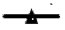 ). On the x-axis is time (hours) and on the y-axis is pressure (psi).

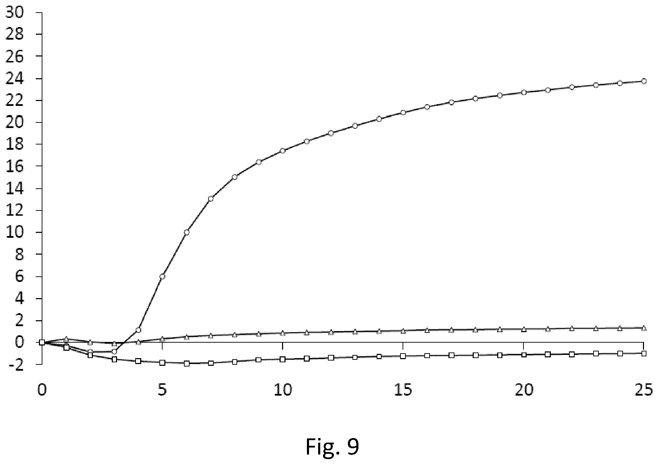

Fig. 9: Cumulative pressure graph of *Bacillus subtilis* strain deposited as DSM32324 ( 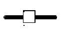 ), *Lactococcus lactis* Strain C ( 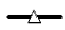 ), and *Clostridium perfringens* type C strain deposited as NCTC3180 ( 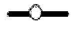 ) during 48 hours incubation. On the x-axis is time (hours) and on the y-axis is pressure (psi).

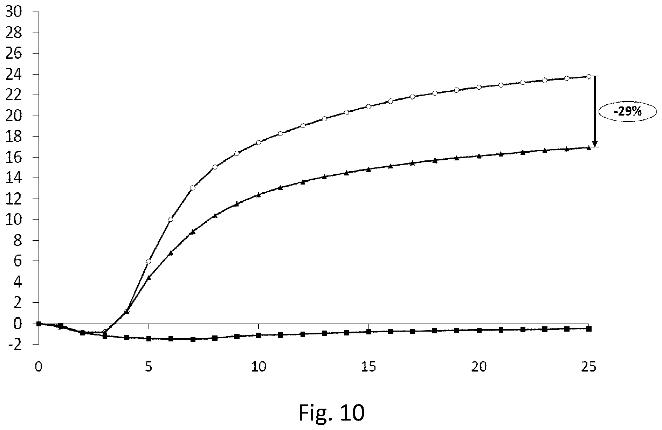

Fig. 10: Cumulative pressure graph of *Clostridium perfringens* type C strain deposited as NCTC3180 during 48 hours incubation as single strain ( 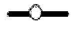 ), together with *Bacillus subtilis* strain deposited as DSM32324 ( 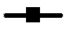 ), and together with *Lactococcus lactis* Strain C ( 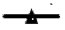 ). On the x-axis is time (hours) and on the y-axis is pressure (psi).

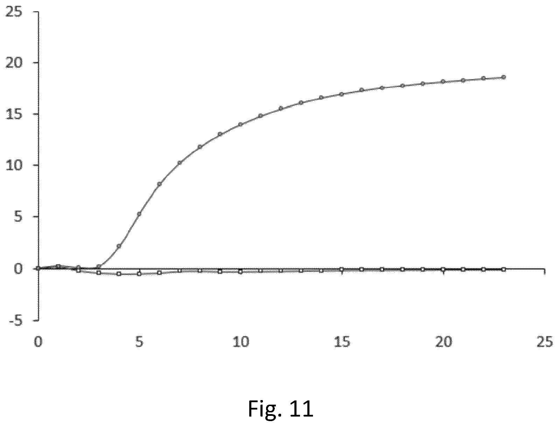

Fig. 11: Cumulative pressure graph of *Bacillus subtilis* strain deposited as DSM17231 ( 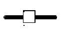 ) and *Clostridium perfringens* type A strain deposited as DSM756 ( 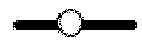 ) during 23 hours incubation. On the x axis is time (hours) and on the y axis is pressure (psi).

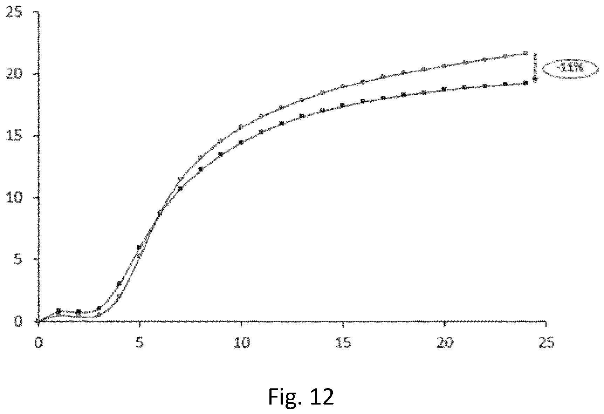

Fig. 12: Cumulative pressure graph of *Clostridium perfringens* type A strain deposited as DSM756 ( 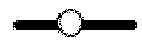 ) during 24 hours incubation as single strain, and together with *Bacillus subtilis* strain deposited as DSM17231 ( 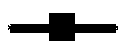 ). On the x axis is time (hours) and on the y axis is pressure (psi).

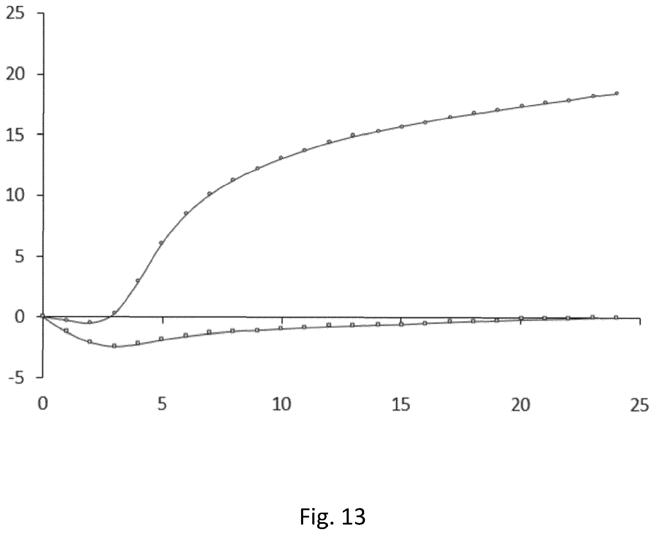

Fig. 13: Cumulative pressure graph of a mixture of *Lactobacillus plantarum* CH6072 strain deposited as DSM16568, *Lactococcus lactis* SR 3.54 strain deposited as NCIMB30117 and *Enterococcus faecium* M74 strain deposited as DSM22502 ( 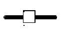 ), and *Clostridium perfringens* type A strain deposited as DSM756 ( 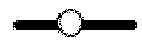 ) during 24 hours incubation. On the x axis is time (hours) and on the y axis is pressure (psi).

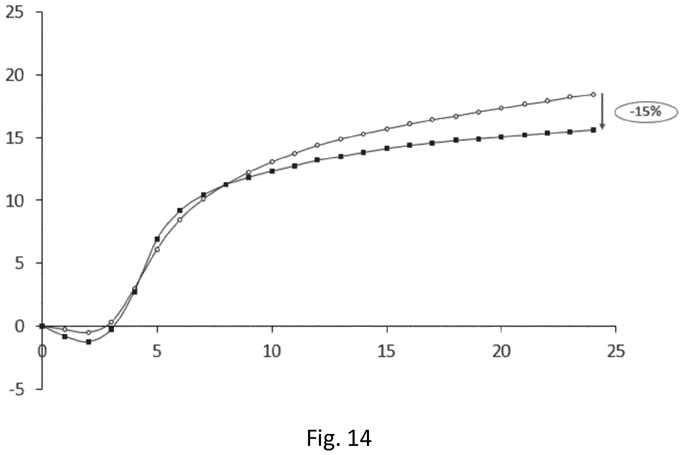

Fig. 14: Cumulative pressure graph of *Clostridium perfringens* type A strain deposited as DSM756 ( 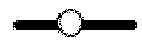 ) during 24 hours incubation as single strain, and together with a mixture of *Lactobacillus plantarum* CH6072 strain deposited as DSM16568, *Lactococcus lactis* SR 3.54 strain deposited as NCIMB30117 and *Enterococcus faecium* M74 strain deposited as DSM22502 ( 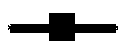 ). On the x axis is time (hours) and on the y axis is pressure (psi).

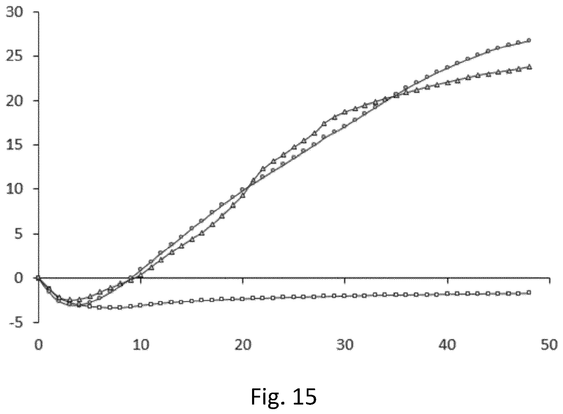

Fig. 15: Cumulative pressure graph of a mixture of *Bacillus subtilis* strain deposited as DSM32324, *Bacillus subtilis* strain deposited as DSM32325 and *Bacillus amyloliquefaciens* strain deposited as DSM25840 ( 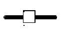 ) during 48 hours incubation. Cumulative gas pressure of *Clostridium septicum* strain deposited as DSM7534 ( 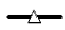 ) and *Clostridium septicum* strain D ( 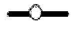 ) during 48 hours incubation. On the x-axis is time (hours) and on the y-axis is pressure (psi).

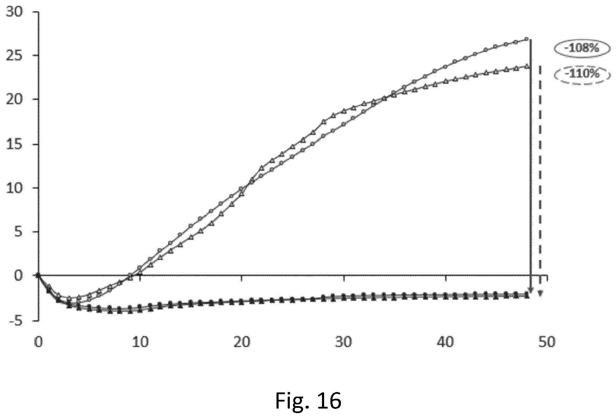

Fig. 16: Cumulative pressure graph of *Clostridium septicum* strain deposited as DSM7534 ( 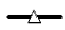 ) during 48 hours incubation as single strain, and together with a mixture of *Bacillus subtilis* strain deposited as DSM32324, *Bacillus subtilis* strain deposited as DSM32325, and *Bacillus amyloliquefaciens* strain deposited as DSM25840 ( 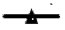 ). Cumulative pressure graph of *Clostridium septicum* strain D during 48 hours incubation as single strain ( 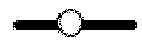 ), and together with a mixture of *Bacillus subtilis* strain deposited as DSM32324, *Bacillus subtilis* strain deposited as DSM32325 and *Bacillus amyloliquefaciens* strain deposited as DSM25840 ( 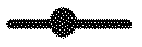 ). On the x-axis is time (hours) and on the y-axis is pressure (psi).

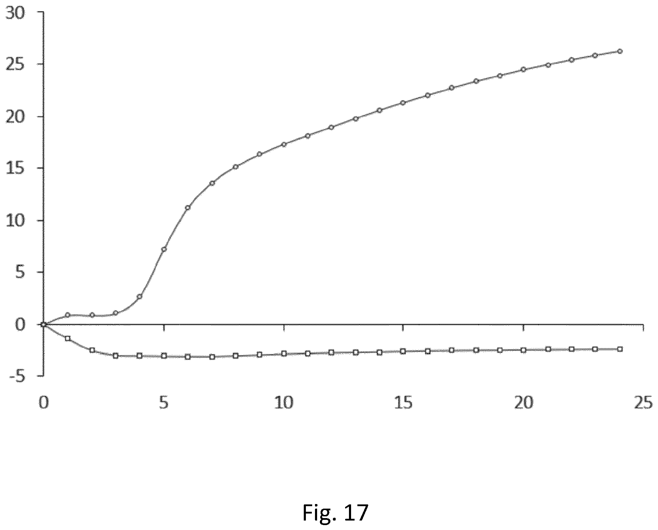

Fig. 17: Cumulative pressure graph of a mixture of *Bacillus subtilis* strain deposited as DSM32324 and *Lactobacillus animalis* strain deposited as PTA-6750 ( 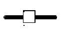 ), and *Clostridium perfringens* type C strain deposited as NTC3180 () 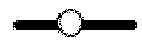 g 24 hours incubation. On the x-axis is time (hours) and on the y-axis is pressure (psi).

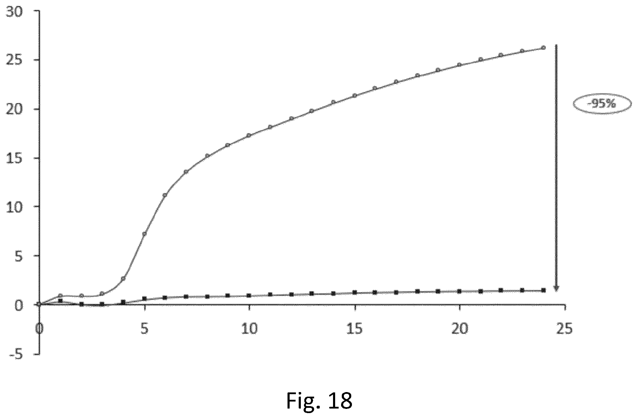

Fig. 18: Cumulative pressure graph of *Clostridium perfringens* type C strain deposited as NTC3180 ( 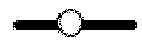 ) during 24 hours incubation as single strain, and together with a mixture of *Bacillus subtilis* strain deposited as DSM32324 and *Lactobacillus animalis* strain deposited as PTA-6750 ( 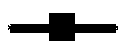 ). On the x-axis is time (hours) and on the y-axis is pressure (psi).

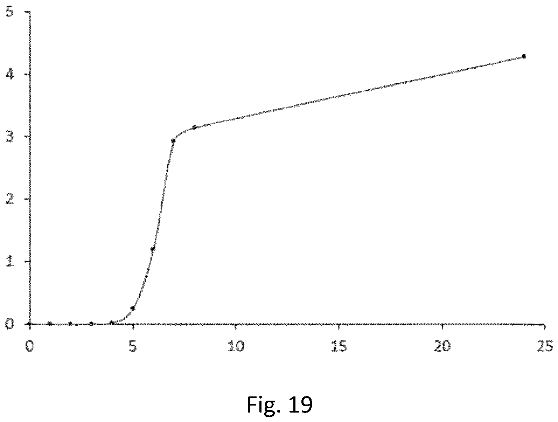

Fig. 19: Growth curve of *Clostridium perfringens* type A strain deposited as DSM756 ( 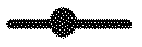 ) in BHI medium. On the x-axis is time (hours) and on the y-axis is optical density (OD₆₀₀ₙₘ).

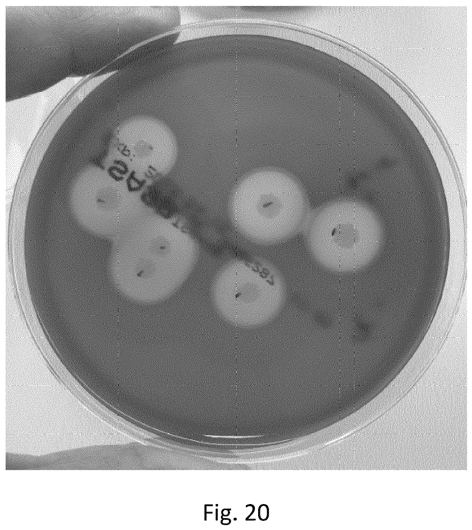

Fig. 20: Colony morphology of *Clostridium perfringens* type C deposited as NTC3180.

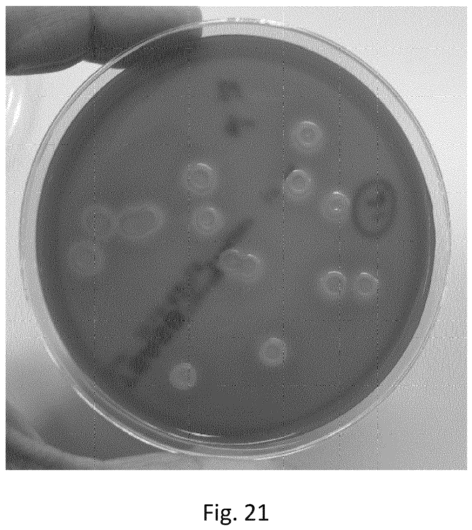

Fig. 21: Colony morphology of *Bacillus subtilis* deposited as DSM32324.

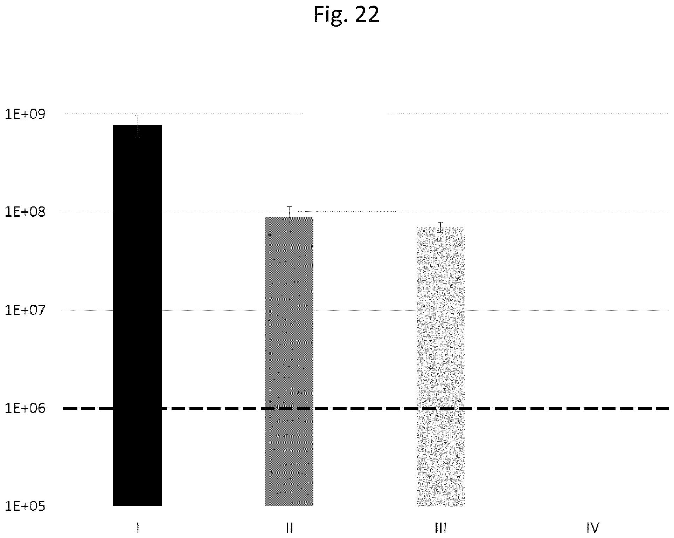

Fig. 22: Enumeration of bottles after run, average CFU/mL in triplicate. I: Bottles inoculated with *Bacillus subtilis* deposited as DSM32324; colonies with *Bacillus* morphology; II: Bottles inoculated with *Clostridium perfringens* type C deposited as NTC3180; colonies with *Clostridium* morphology; III: Bottles inoculated with *Bacillus subtilis* and *Clostridium perfringens;* colonies with *Bacillus* morphology; and IV: Bottles inoculated with *Bacillus subtilis* and *Clostridium perfringens* (same bottles as III); colonies with *Clostridium* morphology. The dotted line is the detection limit.

### DEPOSIT AND EXPERT SOLUTION

The *Bacillus subtilis* strain DSM32324 has been deposited at DSMZ (Deutsche Sammlung von Mikroorganismen und Zellkulturen GmbH, Inhoffenstrasse 7B, D-38124 Braunschweig) on June 8, 2016 by Chr. Hansen A/S, Denmark. The deposit has been made under the conditions of the Budapest Treaty on the International Recognition of the Deposit of Microorganisms for the Purposes of Patent Procedure.

The *Bacillus subtilis* strain DSM32325 has been deposited at DSMZ (Deutsche Sammlung von Mikroorganismen und Zellkulturen GmbH, Inhoffenstrasse 7B, D-38124 Braunschweig) on June 8, 2016 by Chr. Hansen A/S, Denmark. The deposit has been made under the conditions of the Budapest Treaty on the International Recognition of the Deposit of Microorganisms for the Purposes of Patent Procedure.

The *Bacillus subtilis* strain DSM17231 has been deposited at DSMZ (Deutsche Sammlung von Mikroorganismen und Zellkulturen GmbH, Inhoffenstrasse 7B, D-38124 Braunschweig) on April 7, 2005 by Chr. Hansen A/S, Denmark. The deposit has been made under the conditions of the Budapest Treaty on the International Recognition of the Deposit of Microorganisms for the Purposes of Patent Procedure.

The *Bacillus licheniformis* strain DSM17236 has been deposited at DSMZ (Deutsche Sammlung von Mikroorganismen und Zellkulturen GmbH, Inhoffenstrasse 7B, D-38124 Braunschweig) on April 7, 2005 by Chr. Hansen A/S, Denmark. The deposit has been made under the conditions of the Budapest Treaty on the International Recognition of the Deposit of Microorganisms for the Purposes of Patent Procedure.

The *Bacillus amyloliquefaciens* strain DSM25840 has been deposited at DSMZ (Deutsche Sammlung von Mikroorganismen und Zellkulturen GmbH, Inhoffenstrasse 7B, D-38124 Braunschweig) on April 3, 2012 by Chr. Hansen A/S, Denmark. The deposit has been made under the conditions of the Budapest Treaty on the International Recognition of the Deposit of Microorganisms for the Purposes of Patent Procedure.

*The Lactobacillus animalis* strain PTA-6750 has been deposited at American Type Culture Collection (ATCC), USA on May 26, 2005 by Nutrition Physiology Corp., Guymon, Oklahoma, USA. The deposit has been made under the conditions of the Budapest Treaty on the International Recognition of the Deposit of Microorganisms for the Purposes of Patent Procedure and the strain is publicly available as US 8,496,925 referring to the strain has issued.

*The Propionibacterium freudenreichii* strain PTA-6752 has been deposited at American Type Culture Collection (ATCC), USA on May 25, 2005 by Nutrition Physiology Corp., Guymon, Oklahoma, USA. The deposit has been made under the conditions of the Budapest Treaty on the International Recognition of the Deposit of Microorganisms for the Purposes of Patent Procedure and the strain is publicly available as US 7,063,836 referring to the strain has issued.

The *Lactobacillus plantarum* strain DSM16568 has been deposited at DSMZ (Deutsche Sammlung von Mikroorganismen und Zellkulturen GmbH, Inhoffenstrasse 7B, D-38124 Braunschweig) on July 13, 2004 by Chr. Hansen A/S, Denmark. The deposit has been made under the conditions of the Budapest Treaty on the International Recognition of the Deposit of Microorganisms for the Purposes of Patent Procedure.

The *Lactococcus lactis* strain NCIMB30117 is publicly available as Swedish patent SE 511 828 referring to the strain has issued.

The *Enterococcus faecium* strain DSM22502 has been deposited at DSMZ (Deutsche Sammlung von Mikroorganismen und Zellkulturen GmbH, Inhoffenstrasse 7B, D-38124 Braunschweig) on April 22, 2009 by Chr. Hansen A/S, Denmark. The deposit has been made under the conditions of the Budapest Treaty on the International Recognition of the Deposit of Microorganisms for the Purposes of Patent Procedure.

### EXAMPLES

### Materials

Ankom RF gas production system, ANKOM RF Gas Production System (ANKOM Technology, US)
Blue cap bottles (pressure resistant), Duran cat. 218152402
BHI (brain heart infusion) broth, Oxoid, CM225
Blood plates (tryptone soy agar with sheep blood), Oxoid, PB5012A
MRD (maximum recovery diluent), Oxoid, CM0733
TSA plates (tryptone soy agar), Oxoid, PO5012A

The *Clostridium septicum* strain deposited as DSM7534 and the *Clostridium perfringens* type A deposited as DSM756 are type strains and publicly available from DSMZ (Deutsche Sammlung von Mikroorganismen und Zellkulturen GmbH, Inhoffenstrasse 7B, D-38124 Braunschweig).

The *Clostridium perfringens* type C deposited as NCTC3180 is publicly available from Culture Collections, Public Health England, Porton Down, Salisbury, SP4 0JG, UK

### Prior to inoculation

*Bacillus* strains were grown on TSA plates (37°C, aerobic incubation, overnight). A colony of each strain was inoculated in BHI broth and grown overnight (37°C, aerobic), this propagation was used for inoculating the bottles.

*Clostridium* spp. strains were grown on blood plates (37°C, anaerobic incubation, 1-2 days). A colony of each strain was inoculated in BHI broth and grown 1-3 days (37°C, anaerobic).

90 mL of BHI broth was added to 100 mL blue cap bottle (pressure resistant) and autoclaved (121°C, 15 min). Bottles were incubated anaerobically overnight prior to running the experiment.

### Inoculation

Bottles were inoculated with 0.5 mL of propagation of a *Bacillus* strain and 0.5 mL of a *Clostridium* strain. Strains were also inoculated as single strains, here 0.5 mL of sterile water was also added. Bottles were flushed with CO₂ before an Ankom module (head) was securely screwed on the bottle. Bottles were incubated at 37°C. The run was started after placing all the modules in the incubator. For recording gas production, the settings were live interval 50 s, recording interval 10 min, global pressure release 0.75 psi, valve open 250 ms. Cumulative gas production was recorded for 24-72 hours.

Inoculations in examples 1 to 4 were carried out in duplicate and inoculations in example 5 were carried out in triplicate.

### Example 1

| Strain | |
|---|---|
| *Bacillus subtilis* | DSM32324 |
| *Bacillus subtilis* | DSM32325 |
| *Clostridium septicum* | DSM7534 |

The *Bacillus subtilis* strains deposited as DSM32324 and DSM32325 did not produce gas (or very little) during 48h incubation as single strains (Fig. 1).

The total gas production of *Clostridium septicum* deposited as DSM7534 when cultivated as single strain is also shown in Fig. 1.

The *Bacillus subtilis* strain deposited as DSM32324 reduced the gas production from *Clostridium septicum* deposited as DSM7534 by 97% compared to when the *Clostridium septicum* strain was cultivated as single strain (Fig. 2).

The *Bacillus subtilis* strain deposited as DSM32325 reduced the gas production from *Clostridium septicum* deposited as DSM7534 by 96% compared to when the *Clostridium septicum* strain was cultivated as single strain (Fig. 2).

### Example 2

| Strain | |
|---|---|
| *Bacillus subtilis* | DSM32324 |
| *Bacillus licheniformis* | DSM17236 |
| *Clostridium septicum* | DSM7534 |
| *Clostridium septicum* | Strain D |

The *Bacillus subtilis* strain deposited as DSM32324 and the *Bacillus licheniformis* strain deposited as DSM17236 did not produce gas (or very little) during 48h incubation as single strains (Fig. 3).

Two different *Clostridium septicum* strains have been tested and the results in Fig. 3 indicate that the total gas produced during the incubation is strain specific.

The *Bacillus subtilis* strain deposited as DSM32324 inhibited both tested *Clostridium septicum* strains completely (Fig. 4).

The results provided in Fig. 4 indicate that the *Bacillus licheniformis* strain deposited as DSM17236 to a large extent inhibited both tested *Clostridium septicum* strains in that it provided some reduction (68%) of *Clostridium septicum* Strain D (Fig. 4) and a higher reduction (76%) of *Clostridium septicum* deposited as DSM7534.

### Example 3

| Strain | |
|---|---|
| *Lactobacillus animalis* | PTA-6750 |
| *Bacillus subtilis* | Strain A |
| *Bacillus subtilis* | DSM32324 |
| *Clostridium perfringens* type A | DSM756 |

Fig. 5 shows that the *Lactobacillus animalis* strain deposited as PTA-6750, the *Bacillus subtilis* strain A and the *Bacillus subtilis* strain deposited as DSM32324 did not produce gas during incubation. The total gas production of *Clostridium perfringens* type A deposited as DSM756 is also shown in Fig. 5.

The results in Fig. 6 indicate that *Bacillus subtilis* strain deposited as DSM32324 inhibited at least partially (23%) *Clostridium perfringens* type A deposited as DSM756. *Bacillus subtilis* strain A resulted in some reduction of the gas production of *Clostridium perfringens* type A deposited as DSM756 but to a lower extent (6%). *Lactobacillus animalis* deposited as PTA-6750 resulted in 36% reduction of total gas production after incubation with *Clostridium perfringens* type A deposited as DSM756.

### Example 4

| Strain | |
|---|---|
| *Lactobacillus animalis* | PTA-6750 |
| *Propionibacterium freudenreichii* | Strain B |
| *Bacillus subtilis* | DSM32324 |
| *Clostridium perfringens* type C | NCTC3180 |

Fig. 7 shows that *Lactobacillus animalis* deposited as PTA-6750, *Propionibacterium freudenreichii* strain B *and Bacillus subtilis* deposited as DSM32324 did not produce gas during incubation. The total gas production of *Clostridium perfringens* type C deposited as NCTC3180 is also shown in Fig. 7.

The results in Fig. 8 indicate that *Bacillus subtilis* deposited as DSM32324 inhibited *Clostridium perfringens* type C (NCTC3180) completely. *Lactobacillus animalis* deposited as PTA-6750 showed good inhibition by reducing the total gas production of *Clostridium perfringens* type C (NCTC3180) by 74% after incubation. The *Propionibacterium freudenreichii* strain B showed some inhibition by reducing the total gas production of *Clostridium perfringens* type C deposited as NCTC3180 by 27% after incubation.

### Example 5

| Strain | |
|---|---|
| *Bacillus subtilis* | DSM32324 |
| *Lactococcus lactis* | Strain C |
| *Clostridium perfringens* type C | NCTC3180 |

Fig. 9 shows that the *Bacillus subtilis* strain deposited as DSM32324 and the *Lactococcus lactis* strain C when grown as single strains did not produce gas during incubation. The total gas production of *Clostridium perfringens* type C deposited as NCTC3180 is also shown in Fig. 9.

The results in Fig. 10 indicate that *Bacillus subtilis* deposited as DSM32324 inhibited *Clostridium perfringens* type C deposited as NCTC3180 completely and that the *Lactococcus lactis* strain C showed limited reduction of gas production of *Clostridium perfringens* type C deposited as NCTC3180 (29%) after incubation.

### Example 6

| Strain | |
|---|---|
| *Bacillus subtilis* | DSM17231 |
| *Clostridium perfringens* type A | DSM756 |

Fig. 11 shows that the *Bacillus subtilis* strain deposited as DSM17231 did not produce gas during incubation. The total gas production of *Clostridium perfringens* type A deposited as DSM756 is also shown in Fig. 11.

The results in Fig. 12 indicate that *Bacillus subtilis* deposited as DSM17231 resulted in some reduction of gas production of *Clostridium perfringens* type A deposited as DSM756 but to a low extent (11%).

### Example 7

| Strain | |
|---|---|
| *Lactobacillus plantarum* | DSM16568 |
| *Lactococcus lactis* | NCIMB30117 |
| *Enterococcus faecium* | DSM22502 |
| *Clostridium perfringens* type A | DSM756 |

Fig. 13 shows that the mixture of the *Lactobacillus plantarum* strain deposited as DSM16568, the *Lactococcus lactis* strain deposited as NCIMB30117 and the *Enterococcus faecium* strain deposited as DSM22502 did not produce gas during incubation. The total gas production of *Clostridium perfringens* type A deposited as DSM756 is also shown in Fig. 13.

The results in Fig. 14 show that the mixture of *Lactobacillus plantarum* strain deposited as DSM16568, the *Lactococcus lactis* strain deposited as NCIMB30117 and the *Enterococcus faecium* strain deposited as DSM22502 resulted in some reduction of the gas production of *Clostridium perfringens* type A deposited as DSM756 but to a low extent (15%).

### Example 8

| Strain | |
|---|---|
| *Bacillus subtilis* | DSM32324 |
| *Bacillus subtilis* | DSM32325 |
| *Bacillus amyloliquiefaciens* | DSM25840 |
| *Clostridium septicum* | DSM7534 |
| *Clostridium septicum* | Strain D |

Fig. 15 shows that the mixture of the *Bacillus subtilis* strain deposited as DSM32324, the *Bacillus subtilis* strain deposited as DSM32325 and the *Bacillus amyloliquefaciens* strain deposited as DSM25840 did not produce gas during incubation. The total gas production of *Clostridium septicum* deposited as DSM7534 and *Clostridium septicum* strain D is also shown in Fig. 15.

The results in Fig. 16 indicate that the mixture of the *Bacillus subtilis* strain deposited as DSM32324, the *Bacillus subtilis* strain deposited as DSM32325 and the *Bacillus amyloliquefaciens* strain deposited as DSM25840 inhibited completely *Clostridium septicum* deposited as DSM7534 and *Clostridium septicum* strain D.

### Example 9

| Strain | |
|---|---|
| *Bacillus subtilis* | DSM32324 |
| *Lactobacillus animalis* | PTA-6750 |
| *Clostridium perfringens* type C | NTC3180 |

Fig. 17 shows that the mixture of the *Bacillus subtilis* strain deposited as DSM32324 and the *Lactobacillus animalis* strain deposited as PTA-6750 did not produce gas during incubation. The total gas production of *Clostridium perfringens* type C deposited as NTC3180 is also shown in Fig. 17.

The results in Fig. 18 show that the mixture of the *Bacillus subtilis* strain deposited as DSM32324 and the *Lactobacillus animalis* strain deposited as PTA-6750 resulted in reduction of the gas production of *Clostridium perfringens* type C deposited as NTC3180 (95%).

### Example 10

The optical density (OD₆₀₀ₙₘ) of cultures containing *Clostridium perfringens* type A deposited as DSM756 in BHI medium was measured after incubation at 37°C (Fig. 19). The growth curve indicates a correlation with the gas production.

### Example 11

The content of the bottles after a run were enumerated by performing a serial dilution in MRD and spread plating on blood plates from appropriate dilutions. Plates were incubated anaerobically at 37°C overnight. Colonies were counted based on different colony morphologies (see Fig. 20 and 21).

Bottles inoculated with both *Bacillus subtilis* and *Clostridium perfringens* showed a reduction regarding clostridia when enumerated on blood plates (Fig. 22). Compared to the bottles inoculated with *Clostridium perfringens* alone there was a drop of approx. 3 log.

There were no visible clostridia colonies on the plates when enumerating the bottles with the combination of both *Bacillus subtilis* and *Clostridium perfringens.* Based on the plating scheme the level of clostridia was below 1E+06 CFU/mL in the bottles with the combination of strains, indicated with a dotted line on Fig. 22 which is the detection limit.

## Claims

1. A method for quantifying the inhibition efficiency of at least one first bacterial or yeast strain on a single gas producing second bacterial or yeast strain, the method comprising:
a) providing at least one first bacterial or yeast strain which produces no or only negligible volumes of gas;
b) providing a single gas producing second bacterial or yeast strain;
c) cultivating the at least one first bacterial or yeast strain and the single gas producing second bacterial or yeast strain in a closed culture system which is gas impermeable and to which no additional nutrients are added and waste products are not removed;
d) measuring the volume of the total gas produced in the closed culture system of c) over a period of time by means of an automatic gas measuring device measuring the pressure of the gas produced;
e) measuring the volume of the total gas produced by the single gas producing second bacterial or yeast strain when cultivated in absence of any further bacterial or yeast strains in a closed culture system over the same period of time by means of an automatic gas measuring device measuring the pressure of the gas produced; and
f) comparing the pressure of the total gas of d) and e).

2. The method according to claim 1 or 2, wherein when the pressure of the total gas of d) is lower than the pressure of the total gas of e), the at least one first bacterial or yeast strain inhibits the gas producing second bacterial or yeast strain.

3. The method according to any one of the preceding claims, wherein when the pressure of the total gas of d) is equal to or higher than the pressure of the total gas of e), the at least one first bacterial or yeast strain does not inhibit the gas producing second bacterial or yeast strain.

4. The method according to any one of the preceding claims, wherein the closed culture system is an anaerobic culture system.

5. The method according to any one of the preceding claims, wherein the at least one first bacterial or yeast strain is one bacterial or yeast strain.

6. The method according to any one of the preceding claims, wherein the at least one first bacterial or yeast strain is two or more bacterial or yeast strains.

7. The method according to any one of the preceding claims, wherein the at least one first bacterial or yeast strain is a bacterial strain.

8. The method according to any one of the preceding claims, wherein the at least one first bacterial or yeast strain is of the genus *Bacillus.*

9. The method according to any one of the preceding claims, wherein the at least one first bacterial or yeast strain is a lactic acid bacterial strain.

10. The method according to any one of the preceding claims, wherein the at least one first bacterial or yeast strain is of the genera *Lactobacillus* or *Lactococcus.*

11. The method according to any one of the preceding claims, wherein the gas producing second bacterial or yeast strain is a bacterial strain.

12. The method according to any one of the preceding claims, wherein the gas producing second bacterial or yeast strain is of the genus *Clostridium.*

13. The method according to any one of the preceding claims, wherein the gas producing second bacterial or yeast strain is of the species *Clostridium perfringens* or *Clostridium septicum.*

14. The method according to any one of the preceding claims, wherein the method further comprises calculating the percentage of inhibition of the at least one first bacterial or yeast strain on the gas producing second bacterial or yeast strain.

15. A method according to any one of the preceding claims, wherein the method further comprises comparing the inhibitory efficiency of at least two first bacterial or yeast strains with regard to their inhibitory efficiency towards the same single gas producing second bacterial or yeast strain.

## Patentansprüche

1. Verfahren zum Quantifizieren der Hemmwirkung von mindestens einem ersten Bakterien- oder Hefestamm auf einen einzelnen gasproduzierenden zweiten Bakterien- oder Hefestamm, wobei das Verfahren Folgendes umfasst:
a) Bereitstellen mindestens eines ersten Bakterien- oder Hefestamms, der keine oder nur vernachlässigbare Mengen an Gas produziert;
b) Bereitstellen eines einzelnen gasproduzierenden zweiten Bakterien- oder Hefestamms;
c) Kultivieren des mindestens einen ersten Bakterien- oder Hefestamms und des einzelnen gasproduzierenden zweiten Bakterien- oder Hefestamms in einem geschlossenen Kultursystem, das gasundurchlässig ist und dem keine zusätzlichen Nährstoffe zugesetzt und von dem Abfallprodukte nicht entfernt werden;
d) Messen des Volumens des gesamten in dem geschlossenen Kultursystem von c) prozuzierten Gases über einen Zeitraum mittels einer automatischen Gasmessvorrichtung, die den Druck des produzierten Gases misst;
e) Messen des Volumens des gesamten von dem einzelnen gasproduzierenden zweiten Bakterien- oder Hefestamm produzierten Gases, wenn dieser in Abwesenheit weiterer Bakterien- oder Hefestämme in einem geschlossenen Kultursystem über denselben Zeitraum kultiviert wird, mittels einer automatischen Gasmessvorrichtung, die den Druck des produzierten Gases misst; und
f) Vergleichen des Drucks des Gesamtgases von d) und e).

2. Verfahren nach Anspruch 1 oder 2, wobei, wenn der Druck des Gesamtgases von d) niedriger als der Druck des Gesamtgases von e) ist, der mindestens eine erste Bakterien- oder Hefestamm den gasproduzierenden zweiten Bakterien- oder Hefestamm hemmt.

3. Verfahren nach einem der vorhergehenden Ansprüche, wobei, wenn der Druck des Gesamtgases von d) gleich dem oder höher als der Druck des Gesamtgases von e) ist, der mindestens eine erste Bakterien- oder Hefestamm den gasproduzierenden zweiten Bakterien- oder Hefestamm nicht hemmt.

4. Verfahren nach einem der vorhergehenden Ansprüche, wobei das geschlossene Kultursystem ein anaerobes Kultursystem ist.

5. Verfahren nach einem der vorhergehenden Ansprüche, wobei der mindestens eine erste Bakterien- oder Hefestamm ein Bakterien- oder Hefestamm ist.

6. Verfahren nach einem der vorhergehenden Ansprüche, wobei der mindestens eine erste Bakterien- oder Hefestamm zwei oder mehr Bakterien- oder Hefestämme ist.

7. Verfahren nach einem der vorhergehenden Ansprüche, wobei der mindestens eine erste Bakterien- oder Hefestamm ein Bakterienstamm ist.

8. Verfahren nach einem der vorhergehenden Ansprüche, wobei der mindestens eine erste Bakterien- oder Hefestamm der Gattung Bacillusangehört.

9. Verfahren nach einem der vorhergehenden Ansprüche, wobei der mindestens eine erste Bakterien- oder Hefestamm ein Milchsäure-Bakterienstamm ist.

10. Verfahren nach einem der vorhergehenden Ansprüche, wobei der mindestens eine erste Bakterien- oder Hefestamm der Gattung *Lactobacillusoder Lactococcus* angehört.

11. Verfahren nach einem der vorhergehenden Ansprüche, wobei der gasproduzierende zweite Bakterien- oder Hefestamm ein Bakterienstamm ist.

12. Verfahren nach einem der vorhergehenden Ansprüche, wobei der gasproduzierende zweite Bakterien- oder Hefestamm der Gattung *Clostridiumangehört.*

13. Verfahren nach einem der vorhergehenden Ansprüche, wobei der gasproduzierende zweite Bakterien- oder Hefestamm der Art *Clostridium perfringens* oder *Clostridium septicum* angehört.

14. Verfahren nach einem der vorhergehenden Ansprüche, wobei das Verfahren ferner Berechnen des Prozentsatzes der Hemmung des gasproduzierenden zweiten Bakterien- oder Hefestamms durch den mindestens einen ersten Bakterien- oder Hefestamm umfasst.

15. Verfahren nach einem der vorhergehenden Ansprüche, wobei das Verfahren ferner Vergleichen der Hemmwirkung von mindestens zwei ersten Bakterien- oder Hefestämmen im Hinblick auf ihre Hemmwirkung gegenüber demselben einzelnen gasproduzierenden zweiten Bakterien- oder Hefestamm umfasst.

## Revendications

1. Procédé de quantification de l'efficacité d'inhibition d'au moins une première souche bactérienne ou de levure sur une seule seconde souche bactérienne ou de levure productrice de gaz, le procédé comprenant :
a) la fourniture d'au moins une première souche bactérienne ou de levure qui ne produit pas ou seulement des volumes négligeables de gaz ;
b) la fourniture d'une seconde souche bactérienne ou de levure productrice d'un gaz unique ;
c) la culture de l'au moins une première souche bactérienne ou de levure et la seconde souche bactérienne ou de levure productrice d'un gaz unique dans un système de culture fermé qui est imperméable aux gaz et auquel aucun nutriment supplémentaire n'est ajouté et les déchets produits ne sont pas éliminés ;
d) la mesure du volume du gaz total produit dans le système de culture fermé de c) sur une période de temps au moyen d'un dispositif de mesure de gaz automatique mesurant la pression du gaz produit ;
e) la mesure du volume du gaz total produit par la seconde souche bactérienne ou de levure productrice d'un gaz unique lorsqu'elle est cultivée en l'absence de toute autre souche bactérienne ou de levure dans un système de culture fermé pendant la même période de temps au moyen d'un dispositif de mesure de gaz automatique mesurant la pression du gaz produit ; et
f) la comparaison de la pression du gaz total de d) et e).

2. Procédé selon la revendication 1 ou 2, dans lequel lorsque la pression du gaz total de d) est inférieure à la pression du gaz total de e), l'au moins une première souche bactérienne ou de levure inhibe la seconde bactérienne ou souche de levure productrice de gaz.

3. Procédé selon l'une quelconque des revendications précédentes, dans lequel lorsque la pression du gaz total de d) est égale ou supérieure à la pression du gaz total de e), l'au moins une première souche bactérienne ou de levure n'inhibe par la seconde bactérienne ou souche de levure productrice de gaz.

4. Procédé selon l'une quelconque des revendications précédentes, dans lequel le système de culture fermé est un système de culture anaérobie.

5. Procédé selon l'une quelconque des revendications précédentes, dans lequel l'au moins une première souche bactérienne ou de levure est constituée d'une seule souche bactérienne ou de levure.

6. Procédé selon l'une quelconque des revendications précédentes, dans lequel l'au moins une première souche bactérienne ou de levure est constituée de deux souches bactériennes ou de levure ou plus.

7. Procédé selon l'une quelconque des revendications précédentes, dans lequel l'au moins une première souche bactérienne ou de levure est une souche bactérienne.

8. Procédé selon l'une quelconque des revendications précédentes, dans lequel l'au moins une première souche bactérienne ou de levure est du genre *Bacillus.*

9. Procédé selon l'une quelconque des revendications précédentes, dans lequel la au moins une première souche bactérienne ou de levure est une souche bactérienne d'acide lactique.

10. Procédé selon l'une quelconque des revendications précédentes, dans lequel l'au moins une première souche bactérienne ou de levure est des genres *Lactobacillus* ou *Lactococcus.*

11. Procédé selon l'une quelconque des revendications précédentes, dans lequel la seconde souche bactérienne ou de levure productrice de gaz est une souche bactérienne.

12. Procédé selon l'une quelconque des revendications précédentes, dans lequel la seconde souche bactérienne ou de levure productrice de gaz appartient au genre *Clostridium.*

13. Procédé selon l'une quelconque des revendications précédentes, dans lequel la seconde souche bactérienne ou de levure productrice de gaz appartient aux espèces *Clostridium perfringens* ou *Clostridium septicum.*

14. Procédé selon l'une quelconque des revendications précédentes, dans lequel le procédé comprend en outre le calcul du pourcentage d'inhibition d'au moins une première souche bactérienne ou de levure sur la seconde souche bactérienne ou de levure productrice de gaz.

15. Procédé selon l'une quelconque des revendications précédentes, dans lequel le procédé comprend en outre la comparaison de l'efficacité d'inhibition d'au moins deux premières souches bactériennes ou de levure en ce qui concerne leur efficacité d'inhibition vis-à-vis de la même seconde souche bactérienne ou de levure productrice d'un gaz unique.
